# EUROPEAN PATENT APPLICATION

(11) **EP 2 143 340 A1**
(43) Date of publication of application: **13.01.2010**
(21) Application number: 08159810.4
(22) Date of filing: 07.07.2008
(51) Int. Cl.: A23L 1/29, A23L 1/305

(54) **A nutritional composition with free amino acids and structured lipids**

(71) Applicant: Nestec S.A., 1800 Vevey (CH)
(72) Inventor: Frauchiger, Marc Thierry, 3012 Bern (CH); Haschke, Ferdinand, 1814 La Tour-de-Peilz (CH); Magliola, Corinne, 1009 Pully (CH)
(74) Representative: Corticchiato, Olivier

(57) **Abstract**

A nutritional composition is proposed, such as an infant formula composition, that is especially targeted as patients having food allergies or impairments of intestinal absorption. The composition comprises structured lipids. The composition is based on free amino acids and contains a very low amount of peptides or proteins, if any. The composition comprises a source of carbohydrate and has a particular caloric density. The composition can comprise arachidonic acid (ARA) and/or docosahexaenoic acid (DHA). The composition delivers specific nutritional benefits to the patients.

## Description

### Field of the Invention

This invention relates to a nutritional composition with enhanced nutritional value comprising free amino acids. The invention also relates to the use of such composition in the context of infants formula especially for infants suffering from allergies, food allergies, especially cow milk allergies, impaired intestinal absorption and/or conditions of acute or chronic diarrhea. The use of such composition is of particular relevance for low birth weight infants.

### Background to the Invention

Young children and infants are of more fragile health conditions than adults and often more susceptible to various impairments. Such impairments can be transitory and evolve toward normal status or can have a longer term impact on the subjects. Such impairments can be acquired during the first days or months of life or can be present from birth. Genetic heritage from parents plays significant role in such impairments, either by a direct transmission of a genetic deficiency or by the transmission of a predisposition or susceptibility to such impairments. The environment also plays a considerable role, especially in the first days or months of life, by promoting or modulating such impairments directly or synergistically with the genetic susceptibility of the infant.
Typical impairments of the gastric functions are low or mal-absorption of nutrients in the intestinal tract, susceptibility to inflammation, chronic inflammation, allergies or all types, in particular allergies to cow milk, or to lactose.
Symptoms can be difficult to recognize, especially in children and infants, as they can cover a vast array of physiological effects.

Of particular interest in the context of this invention are the impairments of the intestinal absorption and allergies, especially food mediated allergies. In such cases the symptoms can include diarrheas, constipation, irregular stool excretion, abdominal pain, back pains, digestive pain, cramps, cutaneous reaction and /or inflammation, skin redness and the like. Of particular interest are also the digestive impairments and intestinal mal-absorption related to acute sickness, trauma, surgery and other general dysfunctions of the metabolic functions. In the context of the present invention, these are of relevance not only for infants and children but also for adults.

While human milk remains the most ideal nutritional composition for infants, there are instances where infant formulas can be a safe and most appropriate nutritional source.

Infants having a low or very low birth weight birth weight, premature infants, infants with low growth rates, infants presenting pathological syndromes affecting the maturation of their intestinal functions, intestinal development and/or growth are of particular need to such specifically designed infant formulas (and especially of the nutritional composition of the present invention).

Several types of infants formulas exist. Many are based on compositions combining a source of carbohydrate, a source of proteins, a source of lipids as well as other nutrients and micronutrients such as vitamins or minerals. The carbohydrate, in particular of the present invention, can include lactose, be lactose-free and/or include other carbohydrates. The protein source of generally infant formulas can include whey proteins, soy proteins, rice proteins or other animal or vegetal sources of proteins.
Hydrolyzed molecules can help to treat or prevent potential incompatibility between the molecules and infants (or patient in general) with specific needs : for example infant formulas comprising partially or extensively hydrolyzed proteins are well known to be of help for infant having cow milk allergies or premature infants. Similarly partially, extensively or fully hydrolyzed enteral nutritional compositions can be much easier to be absorbed by fragile patients while inducing less or no adverse effects or allergies.

To fight or prevent severe condition like allergies or severe digestive impairments and/or intestinal mal-absorption protein free compositions are known. In those the protein are replaced, totally or substantially by free amino-acids. Usually the balance and composition of the amino acid profile is adjusted to reproduce that found in maternal milk, particularly with respect to essential and semi-essential amino acids. Alternatively the composition can be designed with adjusted levels of specific amino acids, in particular of non essential amino acids, for example in case of metabolic deficiencies for particular amino-acids. Similarly the use of particular amino-acids and their balance can influence the overall taste of the composition.

Similarly lactose-free formulas can help to treat or prevent certain pathological conditions related to the metabolism of lactose. More generally it can be beneficial to provide a specific carbohydrate composition and/or specific digestible or non digestible carbohydrates.

The lipid content of nutritional composition can be adjusted to respond to particular needs or to enhance particular effects. Long-chain polyunsaturated fatty acid, in particular arachidonic acid (ARA) or docosahexaenoic acid (DHA) are sometimes used for their nutritional value.

In that regard the patent publication WO2008016306A1 describes a composition comprising free amino acids and a particular ratio of ARA / DHA.

Generally in infant formulas, the lipid composition can be adjusted in regard to selected molecules or groups of molecules, such as triglycerides. They are generally described as "structured lipids". Triglycerides comprising palmitic acid residues and having a particularly high content of palmitic acids residues in sn2 position have been shown to have beneficial effects.

In that regard the patent publication EP1237419B1 describes, in a context of a formula comprising a mix of proteins and peptides, the use of triglycerides having palmitic acid residues in sn2 position.

Proteins, carbohydrates, lipids, vitamins, minerals, and other complex molecules all interact together to provide a nutritional value to the composition. The complexity of such nutritional matrices is however increased by at least 2 factors : First the desired physiological effect or pathological condition dictates choices for each individual molecular parameter (such as degree of hydrolysis, type of carbohydrates used, absence or presence of certain lipids, % composition of each,...); Second, the molecular parameters (such as the identity of molecules or their quantity in the mix) are not independent from each other but interlocked. Such complex interrelation includes for example contribution of each molecule or group of molecule to the total caloric value of the composition, the amino-acid balance in the composition, the contribution of each ingredient to the overall osmolarity, the functional interactions between the molecules etc,.... . Other interrelations include the bioavailability of the components : one ingredient can reduce or enhance the bioavailability of some other ingredients. This is particularly true for compositions having multiple complex nutrients such as structured lipids and a full range of amino-acids. Taste and stability are other aspects of relevance where some ingredients mixes can have detrimental effects, The positive or negative synergies are multiple and difficult to predict, if not impossible, when considering the intended physiological effects and the potential unintended effects.

There is a need for a nutritional composition that is particularly suitable for patients, infants or adults, that have digestive impairments, in particular intestinal mal-absorption.

There is a need for a nutritional composition for patient, adults or infants, that have allergies, in particular severe allergies and more specifically allergies linked to their food intake.

Similarly there is a need for a composition that helps to treat or prevent inflammations, especially inflammation of the digestive tract or inflammation reactions to food.

There is a need for a nutritional composition that is hypoallergenic and/or easily digestible and that also enhances the fat absorption in the digestive tract and/or enhance the absorption of mineral in particular calcium in the intestinal tract.

There is a need for a nutritional composition that help promote a healthy intestinal transit and/or soften stools and/or prevent or reduce constipation.

There is a need for a composition specially designed for infants, adults or fragile patients suffering from food allergies or impaired intestinal absorption, that enhances the absorption of various nutrients while alleviate their symptoms.

There is a need to maintain and enhance a satisfactory nutrient absorption in fragile patients, particularly those fed enterally, while providing a complete diet.

There is a particular need for low birth weight infants and/or premature infants and/or infants suffering from pathological conditions affecting the maturation or development of their intestinal function or growth.

There is a need for a nutritional composition that combines a number of the above mentioned benefits while being compatible with or easing the cited pathological conditions.

### Summary of the Invention

In a first aspect, the present invention provides for a composition that comprises free amino-acids and which can be protein-free or peptide-free. The composition comprises a carbohydrate source and a lipid source. The lipid source can comprise structured lipids comprising fatty acids triglycerides having an elevated % of palmitic acid residues in the sn2 position of the triglycerides. The lipids source comprises long-chain polyunsaturated fatty acids, preferably ARA and/or DHA.

In a second aspect, the present invention is an infant formula, preferentially intended for infants between 0 and 36 months.

In a third aspect, the present invention comprises also probiotics and/or prebiotics.

In a further aspect the present invention relates to the use of such composition in allergic infants or infants with impaired intestinal absorption, for treating, preventing or alleviating such symptoms while improving calcium absorption in the intestinal tract, and/or improving the fat absorption in the intestinal tract, and/or softening the stool consistency.

### Detailed Description of the Invention

### Definitions: In this specification, the following terms have the following meanings:

"Probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10). The probiotic can comprise a unique strain of micro-organism, of a mix of various strains and/or a mix of various micro-organisms. In case of mixtures the singular term "probiotic" can still be used to designate the probiotic mixture or preparation.

"Prebiotic" generally means a non digestible food ingredient that beneficially affects the host by selectively stimulating the growth and/or activity of micro-organisms present in the gut of the host, and thus attempt to improve host health.

"Sn2 position" refers to the "middle" position of the fatty acid residues in triglycerides, according to standard published nomenclature.

"structured lipids" (SL) are defined, according to general published definitions as lipids that are modified chemically or enzymatically in order to change their structure. Typical structured lipids can include structured triacylglycerols (triglycerides) and structured phospholipids. A typical Triglyceride structured lipid are MLM-type SLs, having medium chain fatty acids (FA) at the 1- and 3- positions (sn1 and sn3) and a long chain fatty acid at the 2- position (sn2 position). Other typical structured lipids contain unsaturated FA at the sn1 and sn3 positions, and a saturated FA at the sn2 position.

For the purpose of this document, % of compositions are expressed as % of dry matter of the composition, unless otherwise indicated. More generally when the "composition" serves as a basis for weight or % calculations, the dry matter value is understood.

For the purpose of this document, the caloric values of amino acids are expressed as the caloric values of the protein equivalents, i.e. the caloric value of a theoretical protein having a similar composition in amino acids.

The weight value of amino acids is understood as the protein equivalent weight value, unless otherwise specified. This represents about 85% of the weight of the individual amino-acids.

The inventors have found that a particular combination of free amino acids, specific triglycerides and long-chain polyunsaturated fatty acids (LC-PUFA) can be of synergistic benefits in patient and/or infants having allergies or impaired intestinal absorption. Its use in such patients and/or infants provides most adequate nutritional benefits.

The inventors have found that its use can promote intestinal calcium absorption and/or fat absorption and/or soften stools in the target group of patients or infants.

The inventors have found that the intestinal absorption can be promoted while preserving a balanced nutritional diet with high bioavailability of the nutrients.

Most importantly the benefits can be delivered without negatively affecting the other nutritional aspects or creating an unbalance in the composition. In particular the organoleptic properties are maintained at an acceptable level while delivering the above mentioned health and nutritional benefits. The composition of the invention, despite its complex composition, is able to sustain stringent manufacturing conditions while preserving high bioavailability, high nutritional benefits and significant biological effects.

Indeed it is known that nutritional compositions are complex matrices in which the sum of the effects of the individual components is hardly predictable. For example, targeting a specific nutritional profile by the inclusion of specific nutrients can impair the stability (for example susceptibility to oxidation) or the organoleptic properties of the composition.

### Target group of patients :

The present invention can be of use for anyone, animals or humans, suffering from the mentioned conditions or at risk of the above mentioned conditions. In particular a specific group that can benefit from the present invention are children up to and including adolescence, and more particularly infants. Infants and children below the age of 36 months, most preferentially infants below the age of 12 months, are of highest relevance for the present invention. Indeed the maturity of the immune system and of the intestinal system for nutrients absorption can be gravely impaired in these groups, temporarily or permanently. For example premature birth can be a factor of enhanced susceptibility to or increased occurrence of the cited pathological conditions. Low birth weight or very low birth weight infants can also be a group of high relevance for the present invention. Infants suffering from pathological conditions affecting the maturation or development of their intestinal function and/or their growth rate are also a group of particular relevance for the present invention. Similarly inherited genotypes can increase susceptibility to the conditions. The susceptibility and frequency of occurrence can also be enhanced in the youngest group and/or the most fragile, by various medical treatments provided to treat other pathologies. The inventors also consider that generally fragile patients are of particular relevance for the present invention: For example, these groups can include (i) adults or children which immune system or inflammation status have been altered by some pathologies or by (ii) some trauma, (iii) patient in intensive care, (iv) elderly patients having fragile immune system and/or elevated inflammation status and (v) elderly patients which intestinal tract is impaired and which exhibit lower than normal intestinal absorption, (vi) patients with severe impairment of the metabolic functions, (vii) patient recovering from surgery, especially gastrointestinal surgery. The present invention can apply to infants, children, adults and elderly subjects.

### Proteins / peptides / amino acids

A protein is a molecule composed of a long chain of amino acids. Small peptides can comprise between 2 and 20 amino acids. Usual protein sources in manufactured nutritional compositions include whey protein, soy proteins, pea proteins, rice proteins and other extract from animal or vegetable origins. The composition of the present invention comprises a very low amount of proteins or peptides, preferably less than 5% or less than 1% of peptides or proteins (as a % nitrogen originating from proteins and peptides over the total nitrogen content). In one embodiment of the invention the composition is substantially free of peptides or proteins. Preferentially the composition comprises less than 1% or less than 0.1 % of peptides or proteins (as a % nitrogen originating from proteins and peptides over the total nitrogen content). Most preferentially the composition comprises 0%, about 0% or only traces of peptides or proteins. In one embodiment the composition of the invention comprises at least 99% of the total amino acids that are in a free form (by weight based on total amino acid content). The "free" form is defined as the monomeric form of the amino-acid. The inventor believe that a very small amount of peptides or proteins (e.g. less than 1% or less than 0.1%; calculated as mentioned above) however do not reduce significantly the benefits of the present invention. A very minimal amount of intact proteins or peptides or no protein or peptides is however most preferred to provides the full benefits of the invention.

The % of amino acids in the composition are calculated on a weight / weight basis over the total amino acids content of the composition (resulting for example from a theoretical complete hydrolysis of all peptides and proteins).

The selection of amino acid is critical in the context of the present invention. In one embodiment the amino acid composition is selected to represent the average composition of amino acids in maternal milk. In another embodiment of the invention the % of Glutamic acid is higher than 0.1% and less than 4.5.% (w/w of total amino acids). Within this range, it is believed that the Glutamic acid influence the taste of the composition in a minimal, i.e. acceptable, manner.

The free amino acids of the present invention comprises between 1g and 4g (expressed as protein equivalent) per 100 kcal of the composition, preferably between 2 g and 3 g. When expressed directly as amino acids weight (not "protein equivalent"), the free amino acids contents of the composition of the present invention can be between 1.2 g and 4.7 g per 100 kcal of composition.

When provided at the cited levels, the free amino acids enable to deliver a composition that is compatible with the cited structured lipids. The composition is stable, especially when prepared with a n emulsifier as cited below, is able to sustain stringent manufacturing processes without affecting its nutrional properties. These benefits are delivered while maintaining a high bioavailability of the structured lipds cited below.

### Lipids

The lipids of the composition of the invention comprise long-chain polyunsaturated fatty acids (LC-PUFA, for which the established general definition applies).

In this document, the % of particular lipids in the composition of the invention are expressed as w/w % out of the total lipids content of the composition.

In one embodiment, the LC-PUFAs (sum of all LC-PUFAs) comprise between 0.05% and 2% (w/w) of the lipids in the composition; preferentially between 0.1% and 0.8%, most preferably between 0.4% and 0.5%.

In one embodiment the composition comprises ARA and/or DHA, preferentially both. In one embodiment the ARA and DHA represent the majority (w/w) of the LC-PUFAs of the composition. In an embodiment the ARA and DHA represent more than 70% or more than 80% (w/w) of the LC-PUFAs of the composition.

In embodiments of the invention, the % amount of ARA is between 0.1% and 0.4% of the lipids in the composition, preferentially between 0.2% and 0.3%. In embodiments of the invention, the % amount of DHA is between 0.1% and 0.4% of the lipids in the composition, preferentially between 0.2% and 0.3%.

In an embodiment both the ARA and DHA are present in the above cited ranges. The ratio between ARA and DHA content (w/w) can be between 3:1 and 1:3, preferably between 2:1 and 1:2, most preferably at 1:1.

In the context of the present invention ARA and the DHA can have a beneficial short and/or long term effect on the reduction of allergies, on the maturation and development of the nervous system and brain, and in the modulation of the immune system.

### Structured lipids :

The lipids of the present invention comprise structured lipids. The structured lipids can comprise fatty acid triglycerides comprising palmitic acid residues. In one embodiment an enhanced proportion of the palmitic acids are in the sn2 position of the triglycerides. Preferably such proportion in sn2 position of between 18% and 50%, or between 30% and 40% of all palmitic acid residues of the glycerides. In one embodiment the palmitic acid residues make up less than 10% (w/w) of all fatty acids residues present in the triglycerides.

Without being bound by the theory but based on the scientific knowledge, it is believed that the palmitic acid residues are generally less absorbed by the intestine as compared to long-chain unsaturated fatty acids. Maintaining a % of palmitic acid equal or less than 10 % of total Fatty acids is hence preferred. Also the palmitic acid residues in sn1 and sn3 positions are less absorbed than these in sn2 position of triglycerides. Released from the positions sn1 and sn3, the palmitic acid residues can form complexes with calcium in the intestine that are not absorbed and enhance constipation. In comparison sufficient palmitic acid in sn2 position is more absorbed and is believed to increase overall fat absorption, increase Calcium absorption and soften the stool consistency.

The % of specific fatty acids are expressed weight / weight as a % of all lipids of the composition.

### Carbohydrates

In one embodiment of the invention the source of carbohydrate used in the composition comprises lactose. In another embodiment however the composition is free of lactose or substantially free of lactose (i.e. less than 5%, less than 1%, or less than 0.1 % of lactose. A composition that is substantially lactose free or with low lactose content has the benefit of reduction / avoiding lactose intolerance symptoms like abdominal pain, flatulence and diarrhea, in patients suffering form lactose intolerance.
In one embodiment the carbohydrates of the invention comprise maltodextrine, glucose syrup or mixture thereof. The maltodextrine can play a significant role in the encapsulation of the lipids components, and hence enhance the stability and facilitate the drying of the composition. In one embodiment the carbohydrates comprise especially modified very low protein (or no protein) potato starch. In one embodiment substantially all carbohydrates come from glucose syrup.

The % of carbohydrates are expressed weight / weight as a % of all carbohydrates of the composition.

In one embodiment of the invention the carbohydrate content is comprised between 8 g and 16 g per 100 kcal of the composition, When the composition of the invention is an infant formula the carbohydrate content is preferably between 9 g and 14g g per 100 kcal of the composition. In one embodiment, the carbohydrates of the composition comprises between 5.0 g and 20 g per 100 kcal of the composition.

The carbohydrate of the invention can include non digestible carbohydrates, in particular prebiotics. Suitable prebiotics include known prebiotic compounds such as carbohydrate compounds selected from the group consisting of inulin, short-chain fructooligosaccharides (FOS), galacto-oligosaccharide (GOS), xylooligosaccharide (XOS), glanglioside, partially hydrolysed guar gum (PHGG) acacia gum, soybean-gum, Lactowolfberry, wolfberry extracts or mixture thereof. Other carbohydrates can be present such as a second carbohydrate acting in synergy with the first carbohydrate and that is selected from the group consisting of xylooligosaccharide (XOS), gum, acacia gum, starch, partially hydrolysed guar gum or mixture thereof.

### Matrix / Caloric value / infant formula

The caloric value of the composition of the invention is between 250 kcal per 100 g of composition (dry matter) and 750 kcal per 100 g of composition (dry matter).

Preferentially, the nutritional composition is an infant formula. In one embodiment it has a caloric value of between 250 and 750 kcal / 100g of composition (dry matter), most preferably having between 450 and 550 kcal / 100 g (dry matter). Such energy value correspond to the optimum caloric values for infants. Various other ranges can be contemplated within the invention as long as they correspond to well established recommended value ranges for the intended patients. In one embodiment the nutritional composition of the invention can be administered by enteral tube feeding or oral feeding, for example for elderly or weak patients.

### Probiotics :

The composition of the present invention can include probiotics or probiotic extracts. It is however essential for the concept underlying the present invention that probiotics do not add an interfering amount of peptides or proteins. The % amount and composition of such probiotics or probiotic extracts should be carefully selected in that respect. It has been found that probiotics can provide specific health benefits while not interfering with the effect of the structured lipds nor of the free amino acids. In certain instances the probiotics can not only be compatible with but also enhance the nutritional benefits of the structured lipids. Also the stability of the entire composition can remain acceptable. The probiotic micro-organisms considered by this invention can include any probiotic selected form the group comprising of Bifidobacterium, Lactobacillus, Streptococcus, Enterococcus and Saccharomyces or mixtures thereof, preferably selected from the group consisting of *Bifidobacterium longum*, *Bifidobacterium lactis*, *Lactobacillus acidophilus, Lactobacillus rhamnosus, Lactobacillus paracasei, Lactobacillus johnsonii, Lactobacillus plantarum, Lactobacillus salivarius, Lactobacillus reuteri, Enterococcus faecium, Streptococcus sp.* and *Saccharomyces boulardii* or mixtures thereof. More preferably the probiotic is selected from the group comprising of *Lactobacillus rhamnosus* CGMCC 1.3724 (nick name NCC4007 and LPR), *Bifidobacterium lactis* CNCM I-3446 sold *inter alia* by the Christian Hansen company of Denmark under the trade mark Bb12 (nick mane NCC2818), *Bifidobacterium longum* ATCC BAA-999 sold by Morinaga Milk Industry Co. Ltd. of Japan under the trade mark BB536, *Lactobacillus paracasei* CNCM I-2116 (nick name NCC2461 and ST11), *Lactobacillus johnsonii* CNCM I-1225 (nick name NCC533 and Lal), *Lactobacillus fermentum* VRI 003 sold by Probiomics (Australia), under the trademark PCC, *Bifidobacterium longum* CNCM 1-2170, *Bifidobacterium longum* CNCM I-2618, *Bifidobacterium breve* sold by Danisco (Denmark) under the trade mark Bb-03, *Bifidobacterium breve* sold by Morinaga (Japan) under the trade mark M-16V and the strain of *Bifidobacterium breve* sold by Institut Rosell (Lallemand) (Canada) under the trade mark R0070, *Lactobacillus paracasei* CNCM I-1292, *Lactobacillus rhamnosus* ATCC 53103 obtainable *inter alia* from Valio Oy of Finland under the trade mark LGG, *Ehterococcus faecium* SF 68, and mixtures thereof. A preferred probiotic is *Lactobacillus rhamnosus* CGMCC 1.3724.

Preferably, the probiotic is present in the composition in an amount equivalent to between 10³ and 10¹⁰ cfu/g of dry composition (cfu = colony forming unit). This expression includes the possibilities that the bacteria are live, inactivated or dead or even present as fragments such as DNA or cell wall materials. In other words, the quantity of bacteria which the formula contains is expressed in terms of the colony forming ability of that quantity of bacteria as if all the bacteria were live irrespective of whether they are, in fact, live, inactivated or dead, fragmented or a mixture of any or all of these states. Preferably the probiotic is present in an amount equivalent to between 10⁴ to 10⁹ cfu/g of dry composition, even more preferably in an amount equivalent to between 10⁶ and 10⁸ cfu/ g of dry composition.

### Other compounds :

The composition of the present invention can include electrolytes, vitamins, minerals, trace elements, taurine, carnitine, choline, inositol and nucleotides..

The compositon of the invention can comprise an emulsifier. The emulsifier is intended to help the encapsulation of the lipid/fat components and hence facilitate the drying of the composition. A typical emulsifier is Citric acid esters of mono and diglycerides of fatty acids (E472c), that can be supplied from Danisco (Copenhagen, Denmark). under the reference GRINDSTED® CITREM. It can be incorporated at between 0.1% to 1.5% of dry matter or at max.level of 9g per liter. Other emulsifiers generally used for enteral compositions or infants formula can be used. The advantages of an emulsifier is of particular relevance for the matrix of the present composition comprising structured lipids and no (or very little) proteins. Indeed the absence of proteins can negatively impact the encapsulation of the lipds, in certain conditions, and can render the presence of an emulsifiers most important.

### Measurement of caloric values and content % :

The measurement of caloric values and % content are made according to standard published methods.

### Example

The following composition, in table 1, is an example of an infant formula composition according to the present invention (in the below table "fat" is taken as a synonym for "lipid"). Optionally an emulsifier can be added to the composition. the composition (GRINDSTED® CITREM from Danisco (Copenhagen, Denmark). The level of emulsifier is selected in the ranges cited above to be compatible with the process conditions.

**Table 1**

| | |
|---|---|
| **Caloric density (kcal/100 ml)** | 70 |
| **Protein Equivalent g/100 kcal** | 2.75 |
| Protein Quality | free Amino Acids only |
| | |
| | |
| **Fat g/100 kcal** | 4.9 |
| **Fat Quality** | Structured lipds (MCT), LC-PUFA, vegetable oils |
| MCT (% fat) | 25 |
| % of the palmitic acid residues in the triglycerides (TG) being the sn2 position. (*) | 34 |
| ARA (% fat) | 0.22 |
| DHA (% fat) | 0.22 |
| | |
| | |
| **Carbohydrates g/100 kcal** | 11.4 |
| **Carbohydrate Quality** | glucose syrup |
| | |
| **Electr.& Min./ 100 kcal** | |
| Na mg | 40 |
| K mg | 115 |
| CI mg | 80 |
| Ca mg | 72 |
| P mg | 48 |
| Ca/P | *1.5* |
| Mg mg | 10 |
| Mn ug | 7.0 |
| | |
| | |
| **Vitamins & TE / 100 kcal** | As per regulatory constraints and/or established nutritional recommendations, in particular : |
| A IU | 400 |
| D IU | 60 |
| E IU | 2.9 |
| K1 ug | 9.9 |
| C mg | 10 |
| B1 mg | 0.079 |
| B2 mg | 0.22 |
| Niacin mg | 1.30 |
| B6 mg | 0.079 |
| Folic acid ug | 11 |
| Pantothenic acid mg | 0.63 |
| B12 ug | 0.3 |
| Biotin ug | 2.6 |
| Choline mg | 9.9 |
| Inositol mg | 5.9 |
| Taurine mg | 7.9 |
| Carnitine mg | 1.6 |
| | |
| **Nucleotides mg/100 kcal** | |
| CMP | 4.6 |
| UMP | 3.0 |
| AMP | 0.5 |
| GMP | 0.3 |

All ingredients are available from common commercial sources.
(*) The structure lipids are provided from Betapol ™ Base that is supplied by Lipid Nutrition (Wormerveer, Netherlands).
The above composition is in a liquid form obtained by diluting 14 g of dry powder composition in 90 ml of water (thus obtaining 100ml of solution).

## Claims

1. -A free amino acids nutritional composition comprising
- at least 99% of free amino acids (by weight based on total amino acids content),
- long chain polyunsaturated fatty acids (LC-PUFA), preferably arachidonic acid (ARA) or Docosahexaenoic acid (DHA), wherein said LC-PUFA are between 0.05% (w/w) and 2% (w/w) of the lipids of said composition, preferably between 0.1 % (w/w) and 0.8 % (w/w),
- an energy density of between 250 kcal and 750 kcal /100g of said composition (dry matter), and wherein
- a source of carbohydrates comprising between 8.0g and 16 g per 100 kcal of said composition,
and wherein said free amino acids comprise between 1g and 4 g (expressed as protein equivalent) per 100 kcal of said composition;.
**characterized in that** said composition further comprises
- structured lipids (SL) comprising fatty acid triglycerides having between 18% and 50%, preferably between 30% and 40%, of the palmitic acid residues in said triglycerides in the sn2 position of said triglycerides.

2. -A infant formula composition of claim 1 wherein said LC-PUFA comprise arachidonic acid (ARA) and docosahexaenoic acid (DHA), wherein the ARA or the DHA or both are between 0.1 % and 0.4% of the lipids of said composition.

3. -A infant formula composition of claim 1 or 2 further comprising a Glutamic acid content of more than 0.1 % and less than 4.5% of said free amino acids (w/w).

4. -The use of a free amino acid nutritional composition according to any of the preceding claims, in food allergic infants or infants with impaired intestinal absorption, for (i) improving calcium absorption in the intestinal tract, (ii) improving fat absorption in the intestinal tract, (iii) softening stool consistency, or combination thereof.

5. -A nutritional composition of any of the preceding claims comprising carbohydrate prebiotics.

6. -A nutritional composition of any of the preceding claims comprising probiotics.

7. -A nutritional composition of any of the preceding claims further comprising :
- Lipids between 4 g and 5 g per 100 kcal of said composition,
- Medium-Chain triglycerides between 20 and 35 % of said lipids,
- Vitamins ,
- Nucleotides between 4 mg and 11 mg per 100 kcal of said composition
- A calcium / phosphorus ration between 1.3 and 2.0
- A positive amount of biotin ,choline, taurine and carnitine,
- Between 5 µg and 20 µg of folic acid per 100kcal of said composition

8. -A nutritional composition any of the preceding claims wherein said composition is an infant formula.

9. -A nutritional composition any of the preceding claims wherein the palmitic acid residues make up less than 10% (w/w) of all fatty acids residues present in said triglycerides.

10. -A nutritional composition any of the preceding claims wherein said carbohydrate comprises maltodextrine, glucose syrup or combination thereof.

11. -A nutritional composition any of the preceding claims wherein said compositon further comprises an emulsifier.
